# EUROPEAN PATENT SPECIFICATION

(11) **EP 3 158 526 B1**
(45) Date of publication and mention of the grant of the patent: **24.08.2022**
(21) Application number: 15809618.0
(22) Date of filing: 17.06.2015
(51) Int. Cl.: G16H 50/30, G16H 50/20, G16H 40/67, G06Q 10/06

(54) **CONTINUOUS MONITORING OF EVENT TRAJECTORIES SYSTEM AND RELATED METHOD**
KONTINUIERLICHE ÜBERWACHUNG EINES SYSTEMS FÜR EREIGNISTRAJEKTORIEN UND ZUGEHÖRIGES VERFAHREN
SURVEILLANCE CONTINUE D'UN SYSTÈME DE TRAJECTOIRES D'ÉVÉNEMENT ET PROCÉDÉ ASSOCIÉ

(30) Priority: 17.06.2014 US 201462013103 P; 12.08.2014 US 201462036285 P
(43) Date of publication of application: 26.04.2017
(60) Divisional application: 22186166.9
(73) Proprietor: University of Virginia Patent Foundation, Charlottesville, VA 22902 (US)
(72) Inventor: MOORMAN, J., Randall, Keswick, VA 22947 (US)
(74) Representative: Grünecker Patent- und Rechtsanwälte PartG mbB
(86) International application number: PCT/US2015/036215
(87) International publication number: WO 2015/195793

(56) References cited:
- US-A1- 2006 116 910
- US-A1- 2007 208 263
- US-A1- 2011 093 249
- US-A1- 2013 231 949
- US-A1- 2013 231 949
- US-A1- 2014 081 092
- US-B1- 7 707 192

## Description

### BACKGROUND

Tools for monitoring and displaying information are commonly used for assessing current functional states of systems. For example, monitors in hospitals are commonly used to collect vitals and other information about a patient's current functional state. However, it may be difficult or time consuming to analyze such data for the purpose of predicting the future state of the system or the patient. US 2013/231949 A1 discloses a risk-based patient monitoring system for critical care patients that combines data from multiple sources to assess the current and the future risks to the patient, thereby enabling providers to review a current patient risk profile and to continuously track a clinical trajectory. The manner of representing clinical trajectory of monitored patient data, such that the magnitude and trajectory of the change is visible via the proposed representation is not disclosed.

### SUMMARY

The invention provides a system according to claim 1 and a computer program product according to claim 8. Preferred embodiments are specified in the dependent claims.

A system for monitoring event trajectories is disclosed. The system includes one or more processors, one or more computer-readable tangible storage devices, and program instructions stored on at least one of the one or more storage devices for execution by at least one of the one or more processors. The program instructions include first program instructions to receive data indicative of a current medical status of a patient. The program instructions further include second program instructions to retrieve data indicative a previous medical status of the patient. The program instructions further include third program instructions to calculate, based on the current medical status and the previous medical status, a trajectory representative of the patient's medical status, the trajectory comprising a magnitude and a direction. The program instructions further include fourth program instructions to communicate the trajectory of the patient's medical status.

A computer program product for monitoring event trajectories is disclosed. The computer program product includes one or more computer-readable tangible storage devices, and program instructions stored on at least one of the one or more storage devices. The program instructions include first program instructions to receive data indicative of a current operational status of a system. The program instructions further include second program instructions to retrieve data indicative a previous operational status of the system. The program instructions further include third program instructions to calculate, based on the current operational status and the previous operational status, a trajectory representative of the system's operational status, the trajectory comprising a magnitude and a direction. The program instructions further include fourth program instructions to communicate the trajectory of the system's operational status.

### BRIEF DESCRIPTION OF THE DRAWINGS

In the accompanying drawings, structures are illustrated that, together with the detailed description provided below, describe embodiments of the invention and explanatory examples.

Like elements are identified with the same reference numerals. It should be understood that elements shown as a single component may be replaced with multiple components, and elements shown as multiple components may be replaced with a single component. The drawings are not to scale and the proportion of certain elements may be exaggerated for the purpose of illustration.
**FIG. 1** illustrates an example display of current values and past values.
**FIG. 2** illustrates an example display of weighted averages of current and past values.
**FIG. 3** illustrates an example display of two representative functions for weighting prior data points to draw a tail.
**FIG. 4** illustrates an example display of two points of a tail.
**FIG. 5** illustrates an example display of a tail.
**FIG. 6** illustrates an example display including bands corresponding to fold-increase in risk.
**FIG. 7** illustrates an example CoMET display for an ICU or hospital ward with several patients.
**FIG. 8** illustrates an example CoMET display of risk of hemorrhage as a function of risk of respiratory decompensation leading to urgent unplanned intubation in Neonatal Intensive Care Unit patients.
**FIG. 9** illustrates a block diagram of an example computing system.

### DETAILED DESCRIPTION

The description outlines, among other things, a display (or output) for use in monitoring complex operational systems using multiple algorithms (and methods) for estimating risk of imminent events in individual components and for assessing status and risk of a multi-component system. It should be appreciated that although the invention and examples referred to herein is of estimating risk of subacute potentially catastrophic illness events in patients in Intensive Care Units and on hospital wards, such display or output may similarly be used other types of systems.

The drawings and description provided in this disclosure relate to displays of bedside monitoring information for use in patient care. A goal is to display both the current status of the patient as well as the trajectory in a simple way.

Values are measured or derived values from monitoring data. For example, these might the entropy or other dynamical measures, or simpler parameters such as the mean and variance. When viewed in a graph format, for example, values may be illustrated as (*x*, y) pairs.

In one example, but not limited thereto, the display shows the current value along with the trajectory. The combined symbol is herein referred to as CoMET. This is achieved by displaying a tail attached to the current (*x, y*) value that represents prior values and the rate of change. This tail requires both direction and magnitude. The direction is given by aligning the current value with the weighted average of prior values. In one example, the more recent values are weighed more heavily than older values. Any suitable weighting function may be used. The magnitude of the tail is calculated from the first (or other) difference between recent points.

Displays, or other suitable types of outputs, may be individual, and appear at every bedside. In one example, displays of data for groups of patients may be useful for identifying patients that are deteriorating quickly.

CoMETs appear in zones that reflect the probability of an imminent catastrophic clinical event. This may be given as a fold-increase in the risk compared to the average. These zones may be represented as risk bands on the display, and offer a means of communicating about patients. For example, "Your patient just went from zone 1 to zone 3" may be displayed.

In one example, three dimensional representations may be constructed to show positions of patients with respect to 3 or more predictive models. Such representation may be realized through standard 3-dimensional ("3-D") plotting, with automated or manual rotation of the cube so that the positions with respect to all 3 axes may be inspected. Alternatively, holographic displays may be used to represent patients in 3-D spaces. As with 2-dimensional plots, an extra dimension of time can be added by showing recent trajectories with programmable window sizes and time steps.

Just as the status of individual patients can be shown in groups to give information about a patient care unit, each unit can be summarized by a single point and trajectory. Thus, groups of a unit shown collectively on a single display may give information about the status of a hospital, or a department of the hospital. This can be used, for example, to assess resource needs for upcoming shifts. Such a representation of the Emergency Department would be used in decision about diverting patients to other hospitals. Such a representation would be useful in assessing the need to call in additional support staff, or to assess the need for emergency supplies. Application of a standard set of predictive models leads to a normalized measure of the degree of severity of the patients and the hospital burden.

It should be appreciated that standard models can be used for more accurate comparisons of the severity of illness in patients, and for better estimation of performance metrics such as the observed to expected mortality ratio. Likewise, outcome measures such as surgical and medical outcomes as a function of diagnosis can be better compared between hospitals when standardized measures of the severity of illness are applied.

In one example, in addition to showing the probabilities of medical diagnoses such as sepsis or hemorrhage, the monitoring may be used to show the results of more discrete medical conditions such as cardiac rhythm. For example, cardiac rhythm may be classified using time series metrics in the time frequency, nonlinear and other mathematical and statistical domains. One approach to synthesizing the results of several such time series measures is to use regression models, each of which reports the likelihood of a particular rhythm. For example, consider classification of rhythms into sinus rhythm, atrial fibrillation, and sinus rhythm with frequent atrial or ventricular ectopy. Each axis of a 3-D representation is the probability of one of these diagnoses. The position of a patient gives the most likely rhythm, and the movement of a patients' results through time can help distinguish between the two states most likely to be confused - atrial fibrillation, which has sudden onset, and frequent ectopy, atrial or ventricular, which is expected to increase and decrease over time.

The shape of the CoMET tail can hold multiple dimensions of data computed from past (x,y) values of the parameters. Curves in the tail can also be computed from past points to represent changing trajectories toward or away from normal. This can be accomplished, for example, by calculating 2 or more tail segments over adjacent recent time periods such as the past 3 hours and the 3 hours before that. The segments can be joined with smoothing techniques for display. The result is more visual information content about the trajectory of the measured parameters over time without adding complexity to the display.

In one example, the display can be dynamic and show progression of CoMET symbols over time to give more information about rates of change in addition to the length and shape of the comet tail.

In one example, multiple predictive models may be summarized by single comets. For example, the highest fold-increase detected among several predictive models may be the only one shown. Its nature can be depicted in any suitable form, such as by its color for example. Thus a patient with a rising risk of hemorrhage as opposed to respiratory decompensation might be represented by a red comet and not a blue one.

In one example, a suitable animation such as blinking may provide further information. For example, an animation may indicate rate of change, may indicate a specific suspected diagnosis, or may indicate another suitable characteristic.

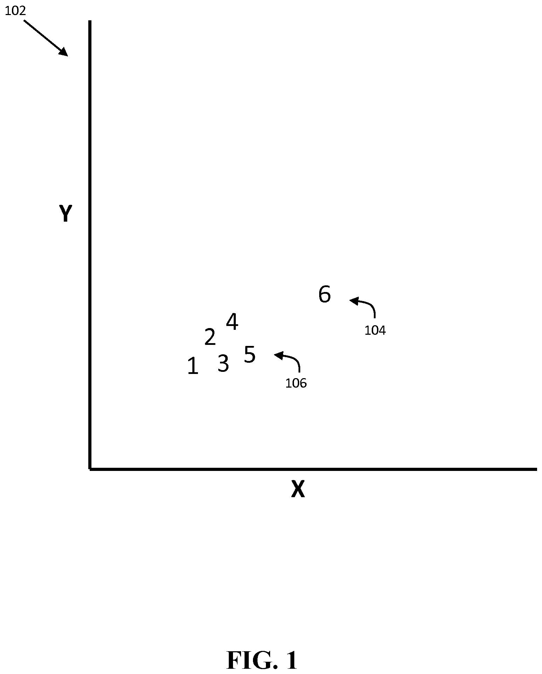

**FIG. 1** illustrates an example display **102** of current values **104** and past values **106.** The ordinate and abscissa are risk estimates using separate prediction algorithms. For example, the ordinate might be the risk of hemorrhage and the abscissa the risk of respiratory decompensation leading to urgent unplanned intubation. Low risk patients appear in the lower left corner; increasing risk is shown by positions at distant points. Here, data points 1 to 5 are sequential previous estimates, and data point 6 is the current value. There is a trend to increasing risk.

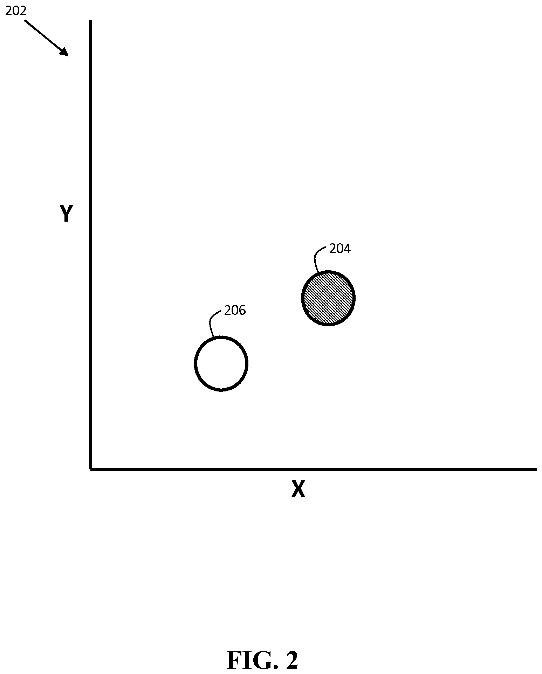

**FIG. 2** illustrates an example display **202** of weighted averages of current **204** and past **206** values. Using the data points from **FIG. 1****,** the weighted average of the prior values is determined. This averaged point is used to portray the trajectory and rate of change of the risk estimates that will be represented by the tail of the CoMET symbol. The current value is represented by the head of the CoMET symbol.

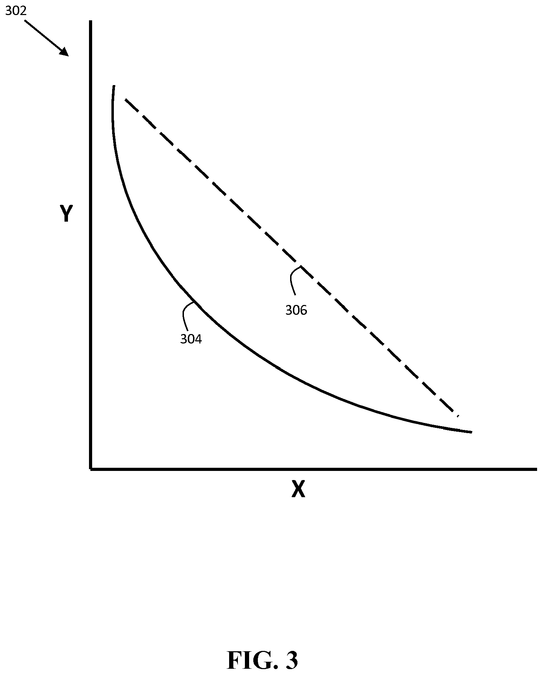

**FIG. 3** illustrates an example display **302** of two representative functions for weighting prior data points to draw a tail. The solid line **304** assigns exponentially declining influence of prior points while the dashed line **306** assigns linearly declining influence. Other functions that assign more or less importance to prior points can be employed.

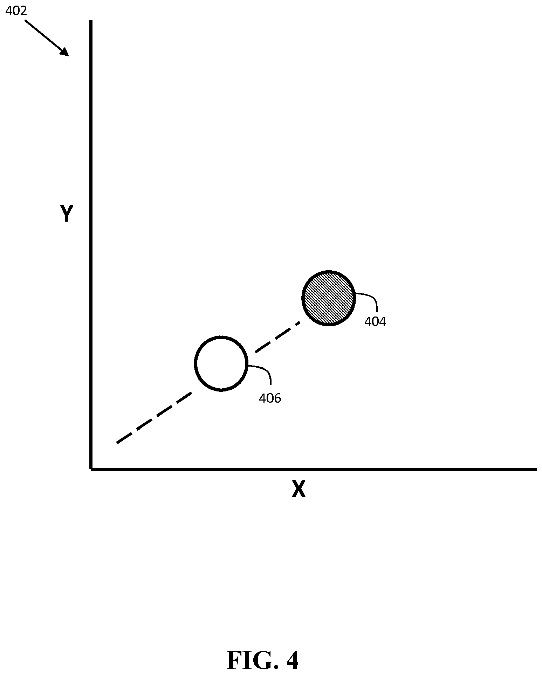

**FIG. 4** illustrates an example display **402** of two points **404** and **406** of a tail. The two points, current value **404** and weighted average of past values **406,** give the direction of the tail. Here, a linear trajectory is shown. Other possibilities include curved trajectories derived from, for example, spline fits through past data points.

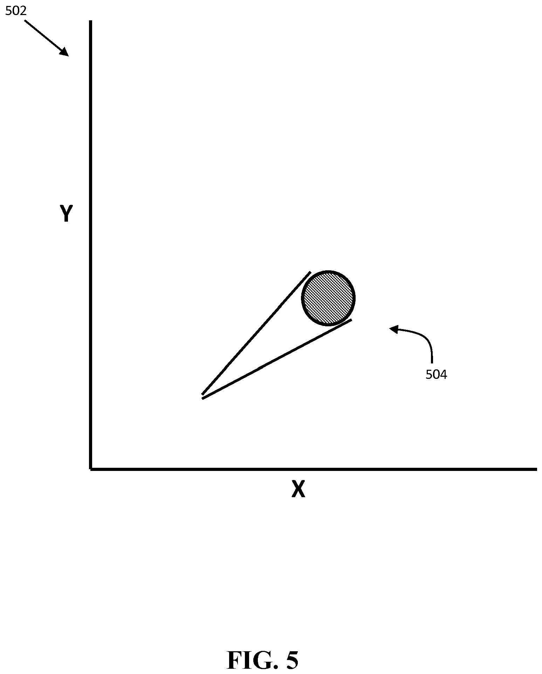

**FIG. 5** illustrates and example display **502** of a tail **504.** The rate of change is reflected in the length of the tail **504.** Fast-moving CoMETs have long tails.

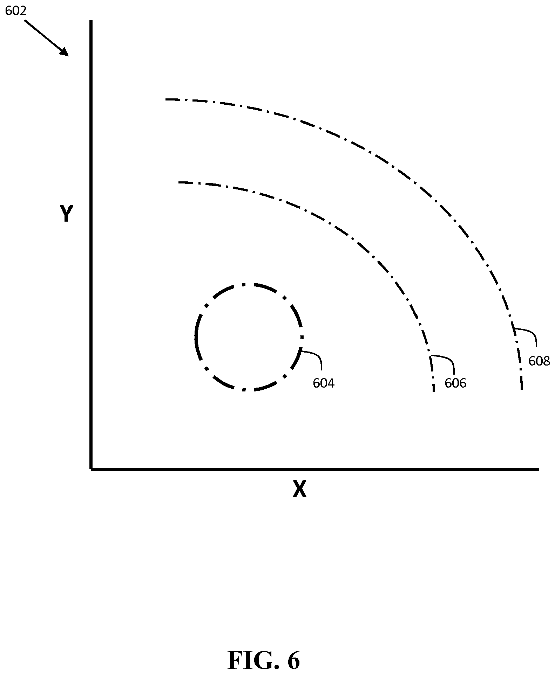

One method of display is to mark the *x,y* plane with bands corresponding to the fold-increase in risk of imminent event. **FIG. 6** illustrates an example display **602** including bands corresponding to fold-increase in risk. In the display **602,** the circle **604** represents the normal, the first arc **606** corresponds to an approximately 2-fold increase in risk, and the second arc **608** corresponds to an approximately 3-fold increase. Other suitable forms of demarcation or coloration might be used to denote the quantitative estimate of risk.

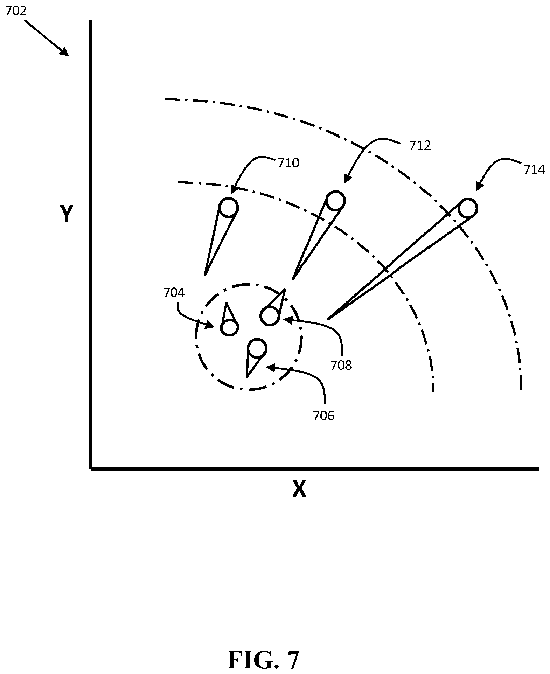

**FIG. 7** illustrates an example CoMET display **702** for an ICU or hospital ward with several patients **704-714.** The information given by the display relates to which patients are stable **704-710** and which are deteriorating **712-714.**

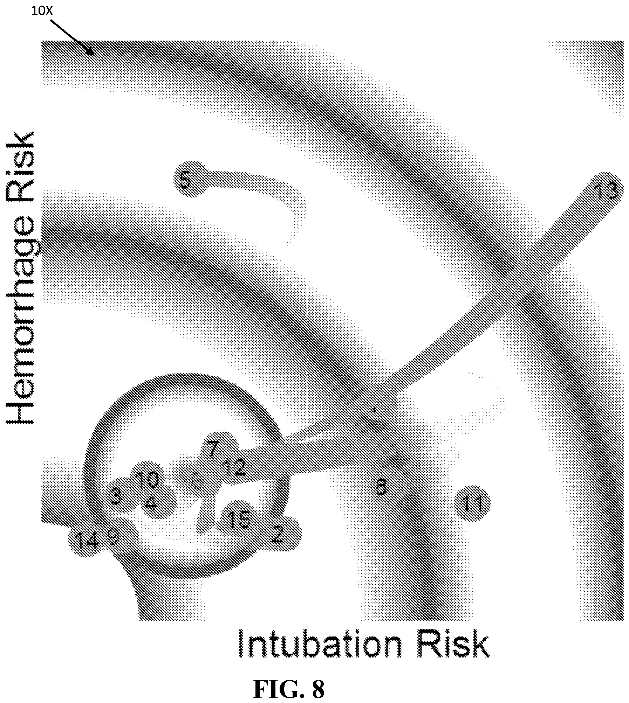

**FIG. 8** illustrates an example CoMET display **802** of risk of hemorrhage as a function of risk of respiratory decompensation leading to urgent unplanned intubation in Neonatal Intensive Care Unit patients. Infants in beds 5, 8, 11 and particularly 13 are perceived to have increased risk of imminent illness, though 8 is improving. The infant in bed 12 is perceived to have rapidly improved risk of imminent illness.

It should be appreciated that the CoMET displays described herein can be customized in suitable ways. For example, the present disclosure provides, among other things, a two-dimensional display of measured or derived parameters that are relevant to a patient's care. One reduction to practice would be to measure at fixed intervals, say, 15 minutes, all the relevant vital signs and lab values. At these intervals, parameters can be calculated on individual or multivariate sets that are relevant to the patient status. Examples include, but are not limited to, the average heart rate, the blood-pressure variability, the cross-correlation of the respiratory rate and heart rate, the entropy of the cardiac inter-beat time series, probabilities of normal and non-normal cardiac rhythms, and multivariate risk assessments calculated from statistical models. A database can be constructed in which each of these measured and derived parameters is recorded for each interval for each patient.

In one example, the user is able to determine interesting groupings of patients. These groupings may be generated by the electronic health record automatically and imported. For these patients, the user may plot any of the measured or derived parameters as a function of any other and use CoMET icons as described elsewhere, with tails that reflect past histories.

In such an example, any physician in the hospital might populate CoMET plots with his or her own patients, and order their rounds by seeing the sickest or most rapidly changing patients first. In addition, patients on a specified geographical unit in the hospital such as a ward or intensive care unit might be enumerated and viewed together. This would be beneficial to health care personnel limited to that unit, such as ICU doctors who might plot hemoglobin concentration as a function of heart rate for early detection of bleeding, cardiologists who might plot troponin level as a function of blood pressure in patients with acute coronary syndromes, and emergency room doctors who seek to triage their often crowded units.

In addition, specialized care centers undertaking acute interventions may improve their outcomes by continuous monitoring for recognized complications. For example, hemodialysis units may provide safer care through continuous display of risk assessment for severe hypotension; cancer chemotherapy infusion centers and sites where immunotherapy is administered might identify severe allergic reactions earlier in their course; and, outside of the hospital setting, blood donation centers may wish for early identification of vasovagal events, heralded by falling heart rate.

Other health care personnel may also identify patients of particular interest for inspection of their data trajectories. For example, pharmacists might elect to view the partial thromboplastin time as a function of hemoglobin concentration in patients receiving heparin infusions. In this way, patients with excess anticoagulation might be detected to be bleeding earlier than ordinary clinical evaluation what allow.

Such functionality arises from a database of all of the measured and derived values for all of the patients in the hospital, with a rendering system capable of importing patient lists and allowing selection of variables to be plotted.

In one example, CoMET head icons can be colored according to the value of the risk estimate or the measured parameter. The (*x,y*) space can be assigned colors according to the values of x and *y,* and a color bar labeled with values can appear to the side. For example, the colors might represent the percentile of the observed value relative to a large legacy database derived from many previous patients. This could be generated by calculating risk estimates or observed parameters from a very large population, and constructing a color isorisk map. Each new calculated or measured parameter is assigned a color based on its percentile ranking in the legacy database. Colors representing low risk may be cooler while those representing higher risk may be warmer, for example. Thus points in the lower left moderate of a plot of one risk estimate as a function of another might have blue heads, and those representing high risk in both would appear in the upper right quadrant and have more red heads.

In one example, measured parameters may be mapped to percentiles or transforms. For CoMET plots of measured values such as vital signs or laboratory tests, linear axes may hide important changes. One reduction to practice is to map measured values to the percentile that they represent in a large legacy database, and to construct the axes as linear scales of percentile from 0 to 100. Intuitively, this allows interpretation of measured values by their extremeness. Another reduction to practice is to transform values to their logarithms, square roots, or by other common arithmetic means to draw attention to changes among low values, and to avoid distortion by extreme outlying high values.

In one example, quadrants of low risk and high risk may be assigned. In the example of one risk estimate as a function of another, the lower left-hand quadrant represents patients at lowest risk and the upper right-hand quadrant represents those at highest risk. The meaning of these quadrants is reversed if the plot is of measured parameters where low values signify clinical risk. For example, the lower left-hand quadrant would represent high risk in a plot of blood pressure as a function of blood count. One reduction to practice would be, in the latter case, to plot the inverse of blood pressure as a function of inverse of blood count. This restores the intuitive interpretation of low risk patients in the lower left hand quadrant and high risk patients in the upper right-hand quadrant.

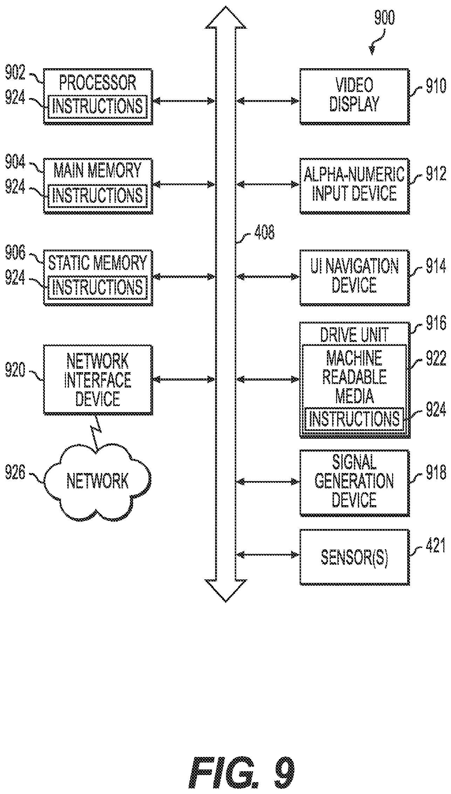

**FIG. 9** illustrates a block diagram of an example computing system **900** that can be used to implement one or more embodiments of the system and method discussed herein.

The computing system **900** can include logic, one or more components, circuits (e.g., modules), or mechanisms. Circuits are tangible entities configured to perform certain operations. In an example, circuits can be arranged (e.g., internally or with respect to external entities such as other circuits) in a specified manner. In an example, one or more computer systems (e.g., a standalone, client or server computer system) or one or more hardware processors (processors) can be configured by software (e.g., instructions, an application portion, or an application) as a circuit that operates to perform certain operations as described herein. In an example, the software can reside (1) on a non-transitory machine readable medium or (2) in a transmission signal. In an example, the software, when executed by the underlying hardware of the circuit, causes the circuit to perform the certain operations.

In an example, a circuit can be implemented mechanically or electronically. For example, a circuit can comprise dedicated circuitry or logic that is specifically configured to perform one or more techniques such as discussed above, such as including a special-purpose processor, a field programmable gate array (FPGA) or an application-specific integrated circuit (ASIC). In an example, a circuit can comprise programmable logic (e.g., circuitry, as encompassed within a general-purpose processor or other programmable processor) that can be temporarily configured (e.g., by software) to perform the certain operations. It will be appreciated that the decision to implement a circuit mechanically (e.g., in dedicated and permanently configured circuitry), or in temporarily configured circuitry (e.g., configured by software) can be driven by cost and time considerations.

Accordingly, the term "circuit" is understood to encompass a tangible entity, be that an entity that is physically constructed, permanently configured (e.g., hardwired), or temporarily (e.g., transitorily) configured (e.g., programmed) to operate in a specified manner or to perform specified operations. In an example, given a plurality of temporarily configured circuits, each of the circuits need not be configured or instantiated at any one instance in time. For example, where the circuits comprise a general-purpose processor configured via software, the general-purpose processor can be configured as respective different circuits at different times. Software can accordingly configure a processor, for example, to constitute a particular circuit at one instance of time and to constitute a different circuit at a different instance of time.

In an example, circuits can provide information to, and receive information from, other circuits. In this example, the circuits can be regarded as being communicatively coupled to one or more other circuits. Where multiple of such circuits exist contemporaneously, communications can be achieved through signal transmission (e.g., over appropriate circuits and buses) that connect the circuits. In embodiments in which multiple circuits are configured or instantiated at different times, communications between such circuits can be achieved, for example, through the storage and retrieval of information in memory structures to which the multiple circuits have access. For example, one circuit can perform an operation and store the output of that operation in a memory device to which it is communicatively coupled. A further circuit can then, at a later time, access the memory device to retrieve and process the stored output. In an example, circuits can be configured to initiate or receive communications with input or output devices and can operate on a resource (e.g., a collection of information).

The various operations of method examples described herein can be performed, at least partially, by one or more processors that are temporarily configured (e.g., by software) or permanently configured to perform the relevant operations. Whether temporarily or permanently configured, such processors can constitute processor-implemented circuits that operate to perform one or more operations or functions. In an example, the circuits referred to herein can comprise processor-implemented circuits.

Similarly, the methods described herein can be at least partially processor-implemented. For example, at least some of the operations of a method can be performed by one or processors or processor-implemented circuits. The performance of certain of the operations can be distributed among the one or more processors, not only residing within a single machine, but deployed across a number of machines. In an example, the processor or processors can be located in a single location (e.g., within a home environment, an office environment or as a server farm), while in other examples the processors can be distributed across a number of locations.

The one or more processors can also operate to support performance of the relevant operations in a "cloud computing" environment or as a "software as a service" (SaaS). For example, at least some of the operations can be performed by a group of computers (as examples of machines including processors), with these operations being accessible via a network (e.g., the Internet) and via one or more appropriate interfaces (e.g., Application Program Interfaces (APIs).)

Example embodiments (e.g., apparatus, systems, or methods) can be implemented in digital electronic circuitry, in computer hardware, in firmware, in software, or in any combination thereof. Example embodiments can be implemented using a computer program product (e.g., a computer program, tangibly embodied in an information carrier or in a machine readable medium, for execution by, or to control the operation of, data processing apparatus such as a programmable processor, a computer, or multiple computers).

A computer program can be written in any form of programming language, including compiled or interpreted languages, and it can be deployed in any form, including as a stand-alone program or as a software module, subroutine, or other unit suitable for use in a computing environment. A computer program can be deployed to be executed on one computer or on multiple computers at one site or distributed across multiple sites and interconnected by a communication network.

In an example, operations can be performed by one or more programmable processors executing a computer program to perform functions by operating on input data and generating output. Examples of method operations can also be performed by, and example apparatus can be implemented as, special purpose logic circuitry (e.g., a field programmable gate array (FPGA) or an application-specific integrated circuit (ASIC)).

The computing system **900** can include clients and servers. A client and server are generally remote from each other and generally interact through a communication network. The relationship of client and server arises by virtue of computer programs running on the respective computers and having a client-server relationship to each other. In embodiments deploying a programmable computing system, it will be appreciated that both hardware and software architectures require consideration. Specifically, it will be appreciated that the choice of whether to implement certain functionality in permanently configured hardware (e.g., an ASIC), in temporarily configured hardware (e.g., a combination of software and a programmable processor), or a combination of permanently and temporarily configured hardware can be a design choice. Below are set out hardware and software architectures that can be deployed in example embodiments.

In an example, the computing system **900** can operate as a standalone device or the computing system **900** can be connected (e.g., networked) to other computing systems.

In a networked deployment, the computing system **900** can operate in the capacity of either a server or a client machine in server-client network environments. In an example, computing system **900** can act as a peer machine in peer-to-peer (or other distributed) network environments. The computing system **900** can be a personal computer (PC), a tablet PC, a set-top box (STB), a Personal Digital Assistant (PDA), a mobile telephone, a web appliance, a network router, switch or bridge, or any machine capable of executing instructions (sequential or otherwise) specifying actions to be taken (e.g., performed) by the computing system **900.** Further, while only a single computing system **900** is illustrated, the term "computing system" shall also be taken to include any collection of computing systems that individually or jointly execute a set (or multiple sets) of instructions to perform any one or more of the methodologies discussed herein.

Example computing system **900** can include a processor **902** (e.g., a central processing unit (CPU), a graphics processing unit (GPU) or both), a main memory **904** and a static memory **906,** some or all of which can communicate with each other via a bus **908.** The computing system **900** can further include a display unit **910,** an alphanumeric input device **912** (e.g., a keyboard), and a user interface (UI) navigation device **911** (e.g., a mouse). In an example, the display unit **910,** input device **917** and UI navigation device **914** can be a touch screen display. The computing system **900** can additionally include a storage device (e.g., drive unit) **916,** a signal generation device **918** (e.g., a speaker), a network interface device **920,** and one or more sensors **921,** such as a global positioning system (GPS) sensor, compass, accelerometer, or other sensor.

The storage device **916** can include a machine readable medium **922** on which is stored one or more sets of data structures or instructions **924** (e.g., software) embodying or utilized by any one or more of the methodologies or functions described herein. The instructions **924** can also reside, completely or at least partially, within the main memory **904,** within static memory **906,** or within the processor **902** during execution thereof by the machine **900.** In an example, one or any combination of the processor **902,** the main memory **904,** the static memory **906,** or the storage device **916** can constitute machine readable media.

While the machine readable medium **922** is illustrated as a single medium, the term "machine readable medium" can include a single medium or multiple media (e.g., a centralized or distributed database, and/or associated caches and servers) that configured to store the one or more instructions **924.** The term "machine readable medium" can also be taken to include any tangible medium that is capable of storing, encoding, or carrying instructions for execution by the machine and that cause the machine to perform any one or more of the methodologies of the present disclosure or that is capable of storing, encoding or carrying data structures utilized by or associated with such instructions. The term "machine readable medium" can accordingly be taken to include, but not be limited to, solid-state memories, and optical and magnetic media. Specific examples of machine readable media can include non-volatile memory, including, by way of example, semiconductor memory devices (e.g., Electrically Programmable Read-Only Memory (EPROM), Electrically Erasable Programmable Read-Only Memory (EEPROM)) and flash memory devices; magnetic disks such as internal hard disks and removable disks; magnetooptical disks; and CD-ROM and DVD-ROM disks.

The instructions **924** can further be transmitted or received over a communications network **926** using a transmission medium via the network interface device **920** utilizing any one of a number of transfer protocols (e.g., frame relay, IP, TCP, UDP, HTTP, etc.). Example communication networks can include a local area network (LAN), a wide area network (WAN), a packet data network (e.g., the Internet), mobile telephone networks (e.g., cellular networks), Plain Old Telephone (POTS) networks, and wireless data networks (e.g., IEEE 802.11 standards family known as Wi-Fi^{®}, IEEE 802.16 standards family known as WiMax^{®}), peer-to-peer (P2P) networks, among others. The term "transmission medium" shall be taken to include any intangible medium that is capable of storing, encoding or carrying instructions for execution by the machine, and includes digital or analog communications signals or other intangible medium to facilitate communication of such software.

The concept of displaying for use in monitoring complex operational systems using multiple algorithms (and methods or techniques) for estimating risk of imminent events in individual components and for assaying status and risk of a multi-component system, may be implemented and utilized with the related processors, networks, computer systems, internet, and components and functions according to the schemes disclosed herein.

While example systems, methods, and so on, have been illustrated by describing examples, and while the examples have been described in considerable detail, it is not the intention to restrict or in any way limit the scope of the appended claims to such detail. It is, of course, not possible to describe every conceivable combination of components or methodologies for purposes of describing the systems, methods, and so on, described herein. Additional advantages and modifications will readily appear to those skilled in the art. Therefore, the invention is not limited to the specific details, and illustrative examples shown or described. Thus, this application is intended to embrace alterations, modifications, and variations that fall within the scope of the appended claims. Furthermore, the preceding description is not meant to limit the scope of the invention. Rather, the scope of the invention is to be determined by the appended claims.

To the extent that the term "includes" or "including" is used in the specification or the claims, it is intended to be inclusive in a manner similar to the term "comprising" as that term is interpreted when employed as a transitional word in a claim. Furthermore, to the extent that the term "or" is employed (e.g., A or B) it is intended to mean "A or B or both." When the applicants intend to indicate "only A or B but not both" then the term "only A or B but not both" will be employed. Thus, use of the term "or" herein is the inclusive, and not the exclusive use. See, Bryan A. Garner, A Dictionary of Modern Legal Usage 624 (2d. Ed. 1995). Also, to the extent that the terms "in" or "into" are used in the specification or the claims, it is intended to additionally mean "on" or "onto." Furthermore, to the extent the term "connect" is used in the specification or claims, it is intended to mean not only "directly connected to," but also "indirectly connected to" such as connected through another component or components.

## Claims

1. A system for monitoring clinical trajectories, the system comprising one or more processors, one or more computer-readable tangible storage devices, and program instructions stored on at least one of the one or more storage devices for execution by at least one of the one or more processors, the program instructions comprising:
first program instructions to receive data indicative of a current medical status of a patient, the current medical status represented by a current value, which is a measured or derived value from patient monitoring data and is represented by a (x, y) pair in a graph format, the axes of the graph representing one of blood pressure, the inverse of blood pressure, blood-pressure variability, heart rate, average heart rate, cross-correlation of the respiratory rate and heart rate, the entropy of the cardiac inter-beat time series, probabilities of normal and non-normal cardiac rhythms, risk of hemorrhage, risk of respiratory decompensation, hemoglobin concentration, troponin level, blood count or the inverse of blood count;
second program instructions to retrieve data indicative of a previous medical status of the patient from patient monitoring data;
third program instructions to calculate, based on the current medical status and the previous medical status, a trajectory representative of the patient's medical status,
wherein the trajectory is represented by a tail attached to the current value in the graph format, the current value and the trajectory appearing in one or more zones reflecting a probability of an imminent clinical event,
wherein the tail represents prior values measured or derived from patient monitoring data and the rate of change, the rate of change being reflected in the length of the tail,
wherein the trajectory includes a magnitude and a direction,
wherein the direction is given by aligning the current value with a weighted average of prior values or derived from fits through past data points,
wherein the magnitude of the trajectory is based on differences in the data indicative of the previous medical status and is calculated from a difference between recent data points; and
fourth program instructions to communicate the trajectory of the patient's medical status.

2. The system of claim 1, wherein the first program instructions receive data indicative of the current medical status from a patient health monitor.

3. The system of claim 1 or 2, wherein the third program instructions calculates weighted average by giving a higher weight to a data value corresponding to a recently obtained medical status over a data value corresponding to a medical status obtained less recently.

4. The system according to any of the preceding claims, wherein the fourth program instructions communicate the trajectory of the patient's medical status to a graph displayed on a user interface.

5. The system according to any of the preceding claims, wherein:
the first program instructions receive data indicative of a current medical status of a plurality of patients;
the second program instructions retrieve data indicative a previous medical status of the plurality of patients;
the third program instructions groups the plurality of patients into a unit and calculate a trajectory representative of the unit's medical status; and
the fourth program instructions communicate the trajectory of the unit's medical status.

6. The system according to any of the preceding claims, wherein the fourth program instructions communicate data indicative of the patient's risk with respect to a clinical event occurring; or
communicate the trajectory of the patient's medical status in a three-dimensional form; or communicate a dynamic trajectory to show progression over time; or
communicate animated data to differentiate the data; or
color-codes the communicated data to indicate different levels of risk of a medical event occurring.

7. The system according to claim 5, wherein the third program instructions groups the plurality of patients into a unit based on a predefined group; or
groups the plurality of patients into a unit based on a user-defined group.

8. A computer program product for monitoring clinical trajectories using the system of claim 1 , the computer program product comprising one or more computer-readable tangible storage devices, and the program instructions of one of the claims 1-7 stored on at least one of the one or more storage devices.

## Patentansprüche

1. System zum Überwachen klinischer Trajektorien, wobei das System einen oder mehr Prozessor/en, eine oder mehr computerlesbare physische Speichervorrichtung/en sowie Programmanweisungen umfasst, die auf wenigstens einer der einen oder mehr Speichervorrichtung/en zur Ausführung durch wenigstens einen von dem einen oder mehr Prozessor/en gespeichert sind, und die Programmanweisungen umfassen:
erste Programmanweisungen, zum Empfangen von Daten, die einen aktuellen medizinischen Status eines Patienten anzeigen, wobei der aktuelle medizinische Status durch einen aktuellen Wert repräsentiert wird, der ein gemessener oder aus Patienten-Überwachungsdaten abgeleiteter Wert ist und durch ein (x, y)-Paar in einem Diagrammformat dargestellt wird, wobei die Achsen des Diagramms Blutdruck, Kehrwert des Blutdrucks, Variabilität des Blutdrucks, Herzfrequenz, durchschnittliche Herzfrequenz, Kreuzkorrelation von Atemfrequenz und Herzfrequenz, die Entropie der Zeitreihe zwischen den Herzschlägen (cardiac inter-beat time series), die Wahrscheinlichkeiten normaler und nicht normaler Herzrhythmen, Blutungsrisiko, Risiko respiratorischer Dekompensation, Hämoglobin-Konzentration, Troponin-Spiegel, Anzahl der Blutkörperchen (blood count) oder den Kehrwert der Anzahl der Blutkörperchen repräsentieren;
zweite Programmanweisungen zum Abrufen von Daten, die einen früheren medizinischen Status des Patienten anzeigen, aus Patienten-Überwachungsdaten;
dritte Programmanweisungen zum Berechnen einer Trajektorie, die für den medizinischen Status des Patienten repräsentativ ist, auf Basis des aktuellen medizinischen Status und des vorherigen medizinischen Status,
wobei die Trajektorie durch einen Rand (tail) dargestellt wird, der an den aktuellen Wert in dem Diagrammformat angehängt ist, und der aktuelle Wert sowie die Trajektorie in einer oder mehr Zone/n erscheinen, die eine Wahrscheinlichkeit eines bevorstehenden klinischen Ereignisses widerspiegelt/widerspiegeln,
der Rand frühere gemessene oder aus Patientenüberwachungsdaten abgeleitete Werte sowie die Änderungsrate darstellt und die Änderungsrate in der Länge des Randes widergespiegelt wird,
die Trajektorie eine Größe und eine Richtung einschließt,
sich die Richtung durch Abgleichen des aktuellen Wertes mit einem gewichteten Durchschnitt früherer Werte ergibt oder aus Verläufen (fits) durch frühere Datenpunkte hindurch abgeleitet wird,
die Größe der Trajektorie auf Unterschieden in den Daten basiert, die den vorherigen medizinischen Status anzeigen, und anhand einer Differenz zwischen aktuellen Datenpunkten berechnet wird;
sowie
vierte Programmanweisungen zum Übermitteln der Trajektorie des medizinischen Status des Patienten.

2. System nach Anspruch 1, wobei die ersten Programmanweisungen Daten, die den aktuellen medizinischen Status anzeigen, von einer Patienten-Gesundheitsüberwachungseinrichtung empfangen.

3. System nach Anspruch 1 oder 2, wobei die dritten Programmanweisungen einen gewichteten Mittelwert berechnen, indem sie einen Datenwert, der einem medizinischen Status entspricht, der vor kurzer Zeit ermittelt wurde, höher gewichten als einen Datenwert, der einem medizinischen Status entspricht, der vor längerer Zeit ermittelt wurde.

4. System nach einem der vorangehenden Ansprüche, wobei die vierten Programmanweisungen die Trajektorie des medizinischen Status des Patienten zu einem Diagramm übermitteln, das an einer Benutzeroberfläche angezeigt wird.

5. System nach einem der vorangehenden Ansprüche, wobei:
die ersten Programmanweisungen Daten empfangen, die einen aktuellen medizinischen Status einer Vielzahl von Patienten anzeigen;
die zweiten Programmanweisungen Daten empfangen, die einen früheren medizinischen Status der Vielzahl von Patienten anzeigen;
die dritten Programmanweisungen die Vielzahl von Patienten zu einer Einheit zusammenfassen und eine Trajektorie berechnen, die für den medizinischen Status der Einheit repräsentativ ist; und
die vierten Programmanweisungen die Trajektorie des medizinischen Status der Einheit übermitteln.

6. System nach einem der vorangehenden Ansprüche, wobei die vierten Programmanweisungen Daten übermitteln, die auf das Risiko des Patienten in Bezug auf das Auftreten eines klinischen Ereignisses hinweisen; oder
die Trajektorie des medizinischen Status des Patienten in einer dreidimensionalen Form übermitteln; oder
eine dynamische Trajektorie übermitteln, die Fortschritt im Laufe der Zeit anzeigt; oder
animierte Daten zum Differenzieren der Daten übermitteln; oder
die übermittelten Daten farblich codieren, um verschiedene Risikostufen für das Auftreten eines medizinischen Ereignisses anzuzeigen.

7. System nach Anspruch 5,
wobei die dritten Programmanweisungen die Vielzahl von Patienten auf Basis einer vordefinierten Gruppe zu einer Einheit zusammenfassen; oder
die Vielzahl von Patienten auf Basis einer benutzerdefinierten Gruppe zu einer Einheit zusammenfassen.

8. Computerprogrammerzeugnis zum Überwachen klinischer Trajektorien unter Einsatz des Systems nach Anspruch 1, wobei das Computerprogrammerzeugnis eine oder mehr computerlesbare physische Speichervorrichtung/en sowie die Programmanweisungen nach einem der Ansprüche 1 - 7 umfasst, die auf wenigstens einer von der einen oder mehr Speichervorrichtung/en gespeichert sind.

## Revendications

1. Système de monitoring de trajectoires cliniques, le système comprenant un ou plusieurs processeurs, un ou plusieurs dispositifs de stockage tangibles lisibles par un ordinateur, et des instructions de programme stockées sur au moins l'un desdits un ou plusieurs dispositifs de stockage pour être exécutées par au moins l'un desdits un ou plusieurs processeurs, les instructions de programme comprenant :
des premières instructions de programme pour recevoir des données indicatives d'un état médical actuel d'un patient, l'état médical actuel étant représenté par une valeur actuelle, qui est une valeur mesurée ou dérivée de données de monitoring du patient et est représentée par une paire (x, y) dans un format de graphe, les axes du graphe représentant un paramètre parmi la pression sanguine, l'inverse de la pression sanguine, la variabilité de la pression sanguine, la fréquence cardiaque, la fréquence cardiaque moyenne, la corrélation croisée entre la fréquence respiratoire et la fréquence cardiaque, l'entropie de la série cardiaque d'intervalles R-R, les probabilités de rythmes cardiaques normaux et anormaux, le risque d'hémorragie, le risque d'une décompensation respiratoire, la concentration d'hémoglobine, le niveau de troponine, la formule sanguine ou l'inverse de la formule sanguine ;
des deuxièmes instructions de programme pour extraire des données indicatives d'un état médical antérieur du patient à partir de données de monitoring du patient ;
des troisièmes instructions de programme pour calculer, sur la base de l'état médical actuel et de l'état médical antérieur, une trajectoire représentative de l'état médical du patient,
dans lequel la trajectoire est représentée par une queue attachée à la valeur actuelle dans le format graphe, la valeur actuelle et la trajectoire apparaissant dans une ou plusieurs zones qui reflètent la probabilité d'un événement clinique imminent,
dans lequel la queue représente des valeurs antérieures mesurées ou dérivées de données de monitoring du patient et le taux de variation, le taux de variation étant reflété par la longueur de la queue,
dans lequel la trajectoire comprend une grandeur et une direction,
dans lequel la direction est donnée en alignant la valeur actuelle avec une moyenne pondérée des valeurs antérieures, ou est dérivée d'ajustements passant par des points de donnée passés,
dans lequel la grandeur de la trajectoire est basée sur des différences dans les données indicatives de l'état médical antérieur et est calculée à partir d'une différence entre des points de donnée récents ; et
des quatrièmes instructions de programme pour communiquer la trajectoire de l'état médical du patient.

2. Système selon la revendication 1, dans lequel les premières instructions de programme reçoivent des données indicatives de l'état médical actuel provenant d'un moniteur de santé du patient.

3. Système selon la revendication 1 ou 2, dans lequel les troisièmes instructions de programme calculent une moyenne pondérée en donnant un poids plus important à une valeur de donnée correspondant à un état médical obtenu récemment qu'à une valeur de donnée correspondant à un état médical obtenu moins récemment.

4. Système selon l'une quelconque des revendications précédentes, dans lequel les quatrièmes instructions de programme communiquent la trajectoire de l'état médical du patient à un graphe affiché sur une interface utilisateur.

5. Système selon l'une quelconque des revendications précédentes, dans lequel :
les premières instructions de programme reçoivent des données indicatives d'un état médical actuel d'une pluralité de patients ;
les deuxièmes instructions de programme extraient des données indicatives d'un état médical antérieur de la pluralité de patients ;
les troisièmes instructions de programme regroupent la pluralité de patients dans une unité et calculent une trajectoire représentative de l'état médical de l'unité ; et
les quatrièmes instructions de programme communiquent la trajectoire de l'état médical de l'unité.

6. Système selon l'une quelconque des revendications précédentes, dans lequel les quatrièmes instructions de programme communiquent des données indicatives du risque pour le patient que se produise un événement clinique ; ou
communiquent la trajectoire de l'état médical du patient sous une forme tridimensionnelle ; ou
communiquent une trajectoire dynamique pour montrer une progression dans le temps ; ou
communiquent des données animées pour différencier les données ; ou
codifient par des couleurs les données communiquées pour indiquer des niveaux différents de risque que se produise un événement médical.

7. Système selon la revendication 5, dans lequel les troisièmes instructions de programme regroupent la pluralité de patients dans une unité sur la base d'un groupe prédéfini ; ou
regroupent la pluralité de patients en une unité sur la base d'un groupe défini par l'utilisateur.

8. Produit logiciel pour le monitoring de trajectoires cliniques en utilisant le système selon la revendication 1, le produit logiciel comprenant un ou plusieurs dispositifs de stockage tangibles lisibles par un ordinateur, et les instructions de programme selon l'une des revendications 1 à 7 stockées sur au moins l'un desdits un ou plusieurs dispositifs de stockage.
